# EUROPEAN PATENT SPECIFICATION

(11) **EP 0 652 862 B1**
(45) Date of publication and mention of the grant of the patent: **30.10.1996**
(21) Application number: 93917638.4
(22) Date of filing: 22.07.1993
(51) Int. Cl.: C07C 231/12, C07C 233/05, C07C 231/02, C07C 231/22, C07C 231/24

(54) **AMIDOACIDS BY ALKALI HYDROLYSIS OF AMIDOESTERS**
AMIDOSÄURE DURCH ALKALINISCHE HYDROLYSIS VON AMIDOESTERN
ACIDES AMIDES OBTENUS PAR L'HYDROLYSE ALCALINE D'ESTERS AMIDES

(30) Priority: 30.07.1992 EP 92202366
(43) Date of publication of application: 17.05.1995
(73) Proprietor: Akzo Nobel N.V., NL-6824 BM Arnhem (NL)
(72) Inventor: BOUWMEESTER, Johannes, Gerhardus, Bernardus, NL-7206 BZ Zutphen (NL); GERRITSEN, Hans, Giovanni, NL-7415 EM Deventer (NL); MEIJER, John, NL-7415 ES Deventer (NL); VAN DOORN, Marcellinus, Alexander, NL-7471 SK Goor (NL); VAN DE BEEK, Theodorus, Wilhelmus, Jozef, NL-7421 EX Deventer (NL); EDELIJN, Herman, Johannes, NL-8017 GA Zwolle (NL)
(74) Representative: Schalkwijk, Pieter Cornelis
(86) International application number: EP9301970
(87) International publication number: WO9403422

(56) References cited:
- EP-A- 0 042 605
- EP-A- 0 170 386
- EP-A- 0 221 387
- EP-A- 0 445 096
- US-A- 3 386 488
- US-A- 4 686 063
- HOUBEN-WEYL 'Methoden der Organischen Chemie, Band E5' 1985 , GEORG THIEME VERLAG , STUTTGART, DE cited in the application see page 225 - page 233

## Description

This invention relates to a process for the preparation of amido acids by hydrolysis of amido esters and to a process for drying such amido acids.

In particular, the present invention relates to a process for the preparation of amido acids of the formula III from amido alkyl esters: 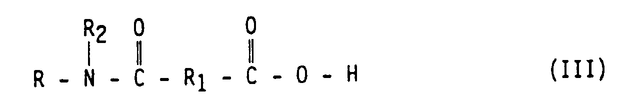 wherein R is selected from C₁₋₂₀ alkyl; R₁ is selected from C₂₋₁₄ alkylene and C₂₋₁₄ alkenylene; and R₂ is selected from hydrogen and alkyl, aryl and aralkyl groups containing 1-10 carbon atoms.

Such amido acids and a method for their preparation are known from European patent application 0 445 096. In the process of this patent application, these amido acids are prepared by the hydrolysis of the amido monoalkyl ester under conditions which avoid transition metal contamination of the amido acids. The hydrolysis process is described as being conventional. While the patent application does not give further details, one can conclude that by conventional is meant an acid hydrolysis process since acid hydrolysis is the industrially preferred method to produce pure acids and in this application an analogous process, namely hydrolysis of diamide by-products of the reaction to produce the monoalkyl ester, is carried out with an inorganic acid. Hydrolysis under alkaline conditions is nowhere suggested.

Production of the amido acids by acid hydrolysis resulted in a low selectivity for the amido acid and, therefore, substantial quantities of undesirable by-products. The formation of these by-products not only reduced the yield of the desired amido acid, but also gave problems in the purification of the amido acid. It was found that this problem could be reduced by continuously removing alkanol from the reactor during the hydrolysis reaction, but such alkanol removal proved to be impractical for large-scale production.

In addition, problems were encountered when drying the amido peracids, particularly when scaling-up the production process. A drying process such as that disclosed in DE 35 39 036, i.e. vacuum drying at 30°C is unsuitable for large scale continuous production due to limitations on throughput rate as well as the requirement for an extremely large drying apparatus.

Accordingly, an alternative process was sought which provides a higher selectivity to the desired amido acid and which does not require the continuous removal of alkanol from the reactor. In addition, an alternative drying method was sought which could be used in large-scale production. These and other objects of the invention will be apparent from the summary and detailed description which follows.

The present invention relates to a process for the alkali hydrolysis of an amido ester to the corresponding amido acid characterized by the steps of:
(A) hydrolyzing, in an aqueous alkaline media, an amido ester represented by the formula I: 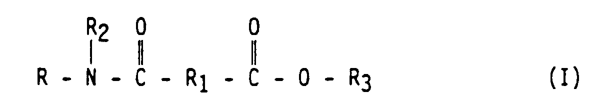 wherein R is selected from C₁₋₂₀ alkyl, R₁ is selected from C₂₋₁₄ alkylene and C₂₋₁₄ alkenylene, and R₂ is selected from hydrogen and alkyl, aryl and aralkyl groups containing 1-10 carbon atoms, and R₃ is selected from C₁₋₈ alkyl; with at least one equivalent of an alkali metal salt per mole of amido ester, at a temperature of 20-160°C to produce an amido acid salt represented by the formula II: 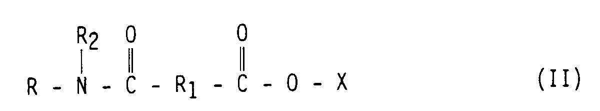 wherein R, R₁, and R₂ are as defined above and X is an alkali metal; and
(B) acidifying the amido acid salt with at least 1.0 equivalents of a strong acid, per mole of amido acid salt, at a temperature of 20-160°C to produce an amido acid represented by the formula III: 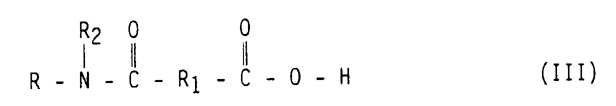 wherein R, R₁, and R₂ are as defined above.

In a second embodiment, the present invention also relates to a process for the drying of an amido acid represented by the formula III, characterized in that the amido acid is dried in a two-stage flash evaporator wherein the first stage operates at 130-140°C, 1.0-2.0 bar and for a time period sufficient to reduce the water content of the amido acid to 1.5%-3.0% by weight, and the second stage operates at 110-135°C, a pressure lower than that used in the first stage, and for a time period sufficient to further reduce the water content of the amido acid to <0.5% by weight. All percentages appearing in this text refer to percent by weight, unless otherwise specified.

European patent application 0 042 685, for example, describes a typical flash evaporation process, such as a falling film evaporator. Milk is evaporated by, for example, evaporating the water therefrom in a series of stages whereby the product from a given stage is flashed into the next stage of the drying process. The final step of this drying process is spray drying.

With respect to the first embodiment of the present invention, it is known from European Patent Application 0 240 138 to prepare cinnamic acid from cinnamic ester by starting the hydrolysis of the ester with an alkali and using water as a solvent in a heterogeneous binary-phase liquid system to obtain an aqueous solution of an alkali cinnamate and then precipitating cinnamic acid by acidifying the aqueous alkaline solution with a mineral acid to maintain the pH at less than 4 in the resulting liquid. However, cinnamic ester lacks an amido functionality and thus is not analogous to the present amido esters.

Japanese Laid-Open number 102614/74 also discloses an alkali hydrolysis process for the hydrolysis of cinnamate esters. This process employs an organic solvent which leads to attendant difficulties in separating the cinnamic acid from the solvent media.

The amido esters used as reactants in the process of the present invention can be obtained by esterifying a polycarboxylic acid of the formula IV: 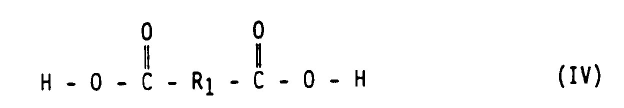 wherein R₁ is as defined above, with a C₁₋₈ alkanol followed by reacting the ester thereby obtained with a monoalkylamine of the formula RNH₂, wherein R is as defined above. Such a process is described in detail in European Patent Application 0 445 096.

Examples of some amido esters which may be employed in the process of the present invention include, but are not limited to, N-n-nonylamido methyl adipate, N-n-octylamido methyl adipate, N-n-decylamido methyl adipate, N-n-nonylamido methyl succinate, N-n-octylamido methyl succinate, N-n-decylamido methyl succinate, as well as the amido esters of the formula I wherein R₃ is methyl, R is octyl, nonyl or decyl, R₁ is trans-C₂ alkenylene and R₂ is hydrogen, and the amido esters wherein R₃ is methyl, R is octyl, decyl, dodecyl or mixtures thereof, R₁ is C₄ alkylene, and R₂ is hydrogen.

The hydrolysis employed in the present invention is an alkali hydrolysis process. In the process, an amido ester of the formula I is reacted with an alkali metal salt in an aqueous alkaline media to produce an alkali metal amido acid salt represented by the formula II. Typically, a molar ratio of amido ester to alkali metal salt of 1-2 is employed, more preferably 1-1.4, and most preferably only a slight excess of alkali metal salt is employed giving a mole ratio of 1-1.1. Of course, sufficient aqueous media must be present for provision of the water necessary for the hydrolysis, as well as to dissolve the amido acid salt.

Alkali metal salts useful in the process of the present invention include, but are not limited to, lithium hydroxide, sodium hydroxide, potassium hydroxide, lithium carbonate, potassium carbonate, sodium carbonate, lithium bicarbonate, potassium bicarbonate, sodium bicarbonate and mixtures thereof. Most preferred of the alkali metal salts are the hydroxides and most particularly, sodium hydroxide.

The hydrolysis step may be carried out at a temperature of 20-160°C. Of course, the lower the temperature, the longer the reaction time. Accordingly, temperatures of 60-100°C are preferred, particularly since these temperatures are often above the melting point of the amido esters, thus easing stirring of the reaction mixture. The most preferred temperature range is 70-95°C which gives a good balance between stirrability, reaction rate and minimization of amido acid salt decomposition. Temperatures above 100°C can be employed so long as a pressure of, for example, 2-6 bar is employed.

The reaction is normally carried out at atmospheric pressure though higher pressures can be employed when using higher reaction temperatures. The reaction time depends upon the reaction temperature and pressure and is preferably in the range of 1 minute to 4 hours. In this regard, a preferred embodiment of the invention is a process whereby at least 99.5% of the amido ester is hydrolyzed to the amido acid salt. Accordingly, in this embodiment, the reaction will be continued for as long as is necessary to achieve this level of conversion.

Vigorous stirring is employed in the process of the invention in order to achieve complete mixing and contact between the alkali metal salt and the amido ester. Alkanols generated in the process of the invention may be recovered by, for example, distillation.

The alkali hydrolysis process of the present invention is advantageous since it can produce substantially 100% conversion of the amido ester to the amido acid while retaining an extremely high selectivity. The corresponding acid hydrolysis, for example, leads to significant quantities of unwanted by-products. Further, in the acid hydrolysis it is necessary to remove the formed alkanol from the reactor during the hydrolysis process in order to achieve a satisfactory yield. Such alkanol removal is impractical for large-scale production. The alkali hydrolysis process of the present invention does not require alkanol removal during the hydrolysis step.

In the second step of the process of the invention, the amido acids can be precipitated by acidifying the resultant aqueous alkaline solution with a strong acid such as mineral acid so that the amido acid can be separated from the reaction mixture by, for example, filtration, decantation, centrifugation, and the like. If precipitation of the amido acid is desired, the acidification step should be carried out at a temperature below the melting point of the amido acid. Such temperatures are typically in the range of about 20-85°C.

Water soluble mineral acids such as, for example, hydrochloric acid, sulfuric acid, phosphoric acid, nitric acid, sodium bisulfate, sodium biphosphate and mixtures thereof may be used in the process. Typically, at least 1.0 equivalents of mineral acid per mole of amido acid salt are employed. More preferably, about 1.0-2.0 equivalents per mole of amido acid salt are used, and, most preferably, 1.0-1.2 equivalents of mineral acid per mole of amido acid salt, are employed. Higher yields of precipitate are obtained by acidification to a pH of about 2-4 to ensure complete precipitation of the amido acid.

In the process of the invention, the mineral acid may be added to the amido acid salt solution or vice versa, or the two materials can be continuously fed to a single reactor. The reaction mixture must be maintained sufficiently fluid to permit effective stirring. This can be ensured by, for example, adding the amido acid salt solution to the mineral acid, or by adding water during the acidification step.

There is no limit on the reaction time for the acidification step except that an inordinately long reaction time, which could result in some acid-catalysed esterification of the amido acid by the alkanol, should be avoided.

The amido acid is then separated from the reaction mixture by filtration, centrifugation or the like, preferably washed with aqueous media which may be acidified water with a pH of 2-4, to remove residual alkanol, amines and alkali metal salts and dried by, for example, air at 20-60°C for up to 24 hours at a pressure of 0.1-1.0 bar in a drying oven. Finally, if desired, the amido acid can be flaked by a drum flaker using conventional means.

A second, more preferred, method for carrying out the acidification step of the present process is to maintain the reaction mixture at a temperature above the melting point of the amido acid. Since the amido acid salt is water soluble and the amido acid is essentially water-insoluble, the result is the formation of a two-layer liquid which can be easily separated. Other than the higher temperature required, the remaining details of the acidification step are the same as for the precipitation method described above.

Typical temperatures for acidification in this manner are 85-160°C and more preferably 90-95°C. Of course the lower limit may vary depending on the melting point of the amido acid. The upper temperature limit is dictated by the tendency of the amido acid to esterify in the presence of strong acid and/or decompose at higher temperatures. One may use temperatures in excess of 160°C, but generally the result will be a lower yield and formation of some undesirable by-products.

Separation of the liquid system is accomplished by removing the alkanol, water and alkali metal salt-containing layer at a temperature above the melting point of the amido acid, preferably 85-110°C, and more preferably 90-95°C. The remaining amido acid-containing layer is preferably washed with an aqueous media such as acidified water of pH of 2-4, to remove substantially all of the residual alkanol therefrom.

The alkanol can be separated from the alkali metal salt by, for example, distillation at a temperature of about 100-105°C and employed or sold as a second product of the reaction.

The obtained amido acid can be flaked and/or dried in any order. In one embodiment, the amido acid is first flaked by a drum flaker and subsequently dried at 20-75°C for a period of up to 24 hours in a drying oven at a low pressure of, for example 0.05-0.5 bar. It is generally desirable to dry the amido acid to a water content of <2.0% and, more preferably, <0.5%.

The flaking operation is a conventional process wherein the material is formed into flakes by a rotating drum flaker which is externally cooled with water to solidify the amido acid.

Another possibility is to first dry the amido acid and then to flake it. This is the most preferred process since it offers the possibilility of drying the amido acid in the melt form, in a relatively short time period and in a manner suitable for large-scale continuous production. Due to the tendency of the amido acid to decompose at higher temperatures, drying of the solid form is generally a long, slow operation.

More particularly, the preferred drying apparatus for drying the molten amido acid is a flash evaporator. The amido acid can be dried to a water content of <2.0% in a single step at, for example, 140-180°C and a pressure of one bar. The dried product may then be flaked in a drum flaker. This single stage drying operation in a flash evaporator provides acceptable results but suffers from the disadvantage that a significant amount of the amido acid decomposes during drying due to the extremely high temperature or long residence time necessary to achieve the desired low water content. For example, in order to dry in ten seconds at atmospheric pressure, a temperature of 160-180°C is needed. At lower temperatures, a significantly lower pressure is required.

It has been found that if the drying operation in the flash evaporator is performed in two distinct stages, it can be accomplished in a short time period and at a temperature such that significantly less amido acid decomposes than in a comparable single stage drying process. More particularly, a first drying step is carried out at a 130-140°C and 1.0-2.0 bar to lower the water content to 1.5-3.0%. Subsequently, a second drying step is carried out at a lower temperature and pressure to further reduce the water content to <0.5%. The second drying stage is generally carried out at a temperature of 110-135°C and a pressure of from 0.05-0.5 bar. The first drying stage generally takes about 10 seconds whereas the second stage takes about 5 seconds.

Though the total residence time is longer than that for the single stage drying, the milder temperatures required apparently result in significantly less decomposition of the amido acid. Of course, minor variations in the temperature, pressure and drying time are possible within the scope of the invention, depending on the total water content of the amido acid coming from the reactor and the particular amido acid being made.

The following examples of the invention are provided for the purpose of illustration and description only and are not to be construed as limiting the scope of the invention in any way. The scope of the invention is to be determined from the claims appended hereto.

### Example 1

A reaction vessel is charged with 136.8 grams of the methylester of the nonylamide of adipic acid and 632 grams of water. This mixture is heated to 80°C and 42.3 grams of a 50% aqueous solution of caustic soda is dosed over a 30 minute period. After a post-reaction time of 15 minutes, the pH of the reaction mixture is adjusted to 3.5 with a 50% aqueous solution of sulfuric acid and the temperature is allowed to rise to 90°C.

The water layer is drained and the organic layer is washed with 320 grams of water. After separation, the organic layer is flaked and dried. The amount of formed nonylamidoadipic acid is 126.5 grams with an assay of 98.5% and having a melting point of 106-107°C.

### Example 2

The process as described in Example 1 is repeated using 228 grams of methylester of the nonylamide of adipic acid, 360 grams of water and 89.6 grams of 50% aqueous solution of caustic soda. This mixture is warmed with stirring to 90-95°C and maintained at this temperature for about 90 minutes (at about 60°C melting of the ester is observed). The reaction mixture is then acidified to pH 3.0 with about 120 grams of 50% aqueous solution of sulfuric acid. The water layer is separated and the organic layer is washed with 150 grams of water over a 15 minute period at 90-93°C. After separation, the organic layer is flaked and dried. In this manner, 215 grams of nonylamidoadipic acid is obtained with an assay of 97.5%.

### Comparative Examples 2A-2B

The process of Example 2 was repeated except that initially 400 g. water was employed and in place of NaOH, 96% sulfuric acid solution (53 g) were used to carry out acid hydrolysis. In Example 2A methanol was removed by distillation during the reaction and an additional 500 grams of water were required. The reaction time was 225 minutes and an assay of 96% was achieved. In Example 2B, steam injection was employed and 1000 g extra water was required. The reaction time was 240 minutes and the assay was only 90%.

### Example 3

The nonylamidoadipic acid obtained by the process of Example 1 was dried in a flash evaporator in two stages. The first stage was carried out at a temperature of 135-140°C, a pressure of 1 bar and for a period of 10 seconds and the second stage was carried out at a temperature of 135-110°C and a pressure of 0.2 bar for a period of 2 seconds. An assay of 98% nonylamidoadipic acid was obtained having a water content of <0.5%.

### Comparative Example 3A

The nonylamidoadipic acid obtained by the process of Example 1 was dried in a flash evaporator in one step at a temperature of 160-180°C, a pressure of 1 bar and for a period of 10 seconds. A yield of 96.5% nonylamidoadipic acid was obtained having a water content of <0.5%.

Example 3 and Comparative Example 3A demonstrate the unexpected advantages of the preferred drying process of the present invention, namely that it can be carried out in seconds on a large-scale and without significant decomposition of the amido acid.

## Claims

1. A process for the alkali hydrolysis of an amido ester to produce an amido acid characterized by the steps of:
(A) hydrolyzing, in an aqueous alkaline media, an amido ester represented by the formula I: 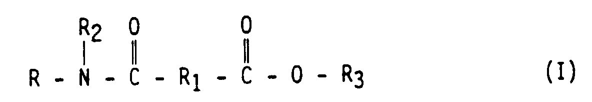 wherein R is selected from C₁₋₂₀ alkyl, R₁ is selected from C₂₋₁₄ alkylene and C₂₋₁₄ alkenylene, R₂ is selected from hydrogen and alkyl, aryl and aralkyl groups containing 1-10 carbon atoms and R₃ is selected from C₁₋₈ alkyl; with at least one equivalent of an alkali metal salt per mole of amido ester at 20-160°C to produce an amido acid salt; and
(B) acidifying the amido acid salt with at least 1.0 equivalents of a strong acid, per mole of amido acid salt, at 20-160°C to produce an amido acid.

2. A process in accordance with claim 1 wherein hydrolysis step (A) is carried out until >99.5%, of the amido ester is converted to the corresponding amido acid salt.

3. A process in accordance with any one of claims 1-2 further comprising the steps of:
(C) precipitating the amido acid from the aqueous reaction media of step (B),
(D) isolating the amido acid solid precipitate,
(E) washing the amido acid solid precipitate with a sufficient amount of an aqueous media to remove substantially all residual alkanol therefrom, and
(F) drying the washed amido acid solid precipitate at a temperature of 20-95°C.

4. A process in accordance with any one of claims 1-2 wherein step (B) is carried out at a temperature above the melting point of the amido acid, and further comprising the steps of:
(C) separating the liquid phase containing the amido acid at a temperature above the melting point of the amido acid, and
(D) washing the liquid phase containing the amido acid with a sufficient amount of an aqueous media to remove substantially all residual alkanol therefrom.

5. A process in accordance with claim 4 further comprising the steps of:
(E) drying and flaking the amido acid, characterized in that the drying and flaking steps may be performed in any order and that the drying step reduces the water content of the amido acid to <2.0%, by weight.

6. A process in accordance with claim 5 wherein the drying step is performed prior to the flaking step and the drying step comprises drying the amido acid in a flash evaporator at 110-180°C.

7. A process in accordance with claim 6 wherein the drying step is performed in a two-stage flash evaporator, the first stage operating at 130-140°C, 1.0-2.0 bar and for a time period sufficient to reduce the water content of the amido acid to 1.5-3.0% by weight, and the second stage operating at a lower temperature and pressure than was used in the first stage and for a time period sufficient to further reduce the water content of the amido acid to <0.5% by weight.

## Patentansprüche

1. Verfahren zur Alkalihydrolyse eines Amidoesters zur Erzeugung einer Amidosäure, gekennzeichnet durch die Schritte:
(A) Hydrolysieren in einem wässrigen alkalischen Medium eines Amidoesters entsprechend der Formel I: 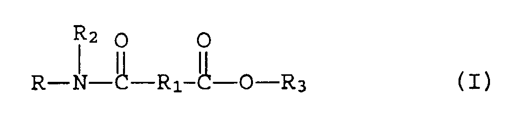 worin R gewählt ist aus C₁₋₂₀ Alkyl, R₁ gewählt ist aus C₂₋₁₄ Alkylen und C₂₋₁₄ Alkenylen, R₂ gewählt ist aus Wasserstoff und Alkyl-, Aryl- und Aralkylgruppen, die 1-10 Kohlenstoffatome enthalten, und R₃ gewählt ist aus C₁₋₈ Alkyl, mit mindestens einem Äquivalent eines Alkalimetallsalzes pro Mol Amidoester bei 20-160°C zur Bildung eines Amidosäuresalzes und
(B) Ansäuern des Amidosäuresalzes mit mindestens 1.0 Äquivalenten einer starken Säure pro Mol des Amidosäuresalzes bei 20-160°C zur Erzeugung einer Amidosäure.

2. Verfahren nach Anspruch 1, bei welchem der Hydrolyseschritt (A) durchgeführt wird, bis >99.5% des Amidoesters in das entsprechende Amidosäuresalz umgewandelt sind.

3. Verfahren nach einem der Ansprüche 1-2 mit den zusätzlichen Schritten:
(C) Fällen der Amidosäure aus dem wässrigen Reaktionsmedium von Schritt (B),
(D) Isolieren des festen Amidosäureniederschlags,
(E) Waschen des festen Amidosäureniederschlags mit einem ausreichenden Anteil eines wässrigen Mediums zur Entfernung praktisch des gesamten restlichen Alkanols, und
(F) Trocknen des gewaschenen festen Amidosäureniederschlags bei einer Temperatur von 20-95°C.

4. Verfahren nach einem der Ansprüche 1-2, bei dem der Schritt (B) bei einer Temperatur über dem Schmelzpunkt der Amidosäure durchgeführt wird, und mit dem zusätzlichen Schritten:
(C) Abtrennen der die Amidosäure enthaltenden flüssigen Phase bei einer Temperatur über dem Schmelzpunkt der Amidosäure und
(D) Waschen der die Amidosäure enthaltenden flüssigen Phase mit einem ausreichenden Anteil eines wässrigen Mediums zur Entfernung praktisch des gesamten restlichen Alkanols.

5. Verfahren nach Anspruch 4 mit dem zusätzlichen Schritt: (E) Trocknen und Flocken der Amidosäure, dadurch gekennzeichnet, dass die Trocknungs- und Flockungsschritte in jeder Reihenfolge durchgeführt werden können und dass der Trocknungsschritt den Wassergehalt der Amidosäure auf <2.0 Gew.% vermindert.

6. Verfahren nach Anspruch 5, bei welchem der Trocknungsschritt vor dem Flockungsschritt durchgeführt wird und der Trocknungsschritt das Trocknen der Amidosäure in einem Schnellverdampfer bei 110-180°C umfasst.

7. Verfahren nach Anspruch 6, bei dem der Trocknungsschritt in einem Zweistufen-Schnellverdampfer durchgeführt wird, wobei die erste Stufe bei 130-140°C, 1.0-2.0 bar und während eines Zeitraumes durchgeführt wird, der ausreicht, um den Wassergehalt der Amidosäure auf 1.5-3.0 Gew.% zu senken, und die zweite Stufe bei einer niedrigeren Temperatur und einem niedrigeren Druck durchgeführt wird, als in der ersten Stufe, und zwar während einer Zeitspanne, die ausreicht, um den Wassergehalt der Amidosäure auf <0.5 Gew.% zu vermindern.

## Revendications

1. Un procédé d'hydrolyse alcaline d'un ester amide pour obtenir un acide amide, caractérisé par les étapes de:
(A) hydrolyse, en milieu alcalin aqueux, d'un ester amide répondant à la formule I: 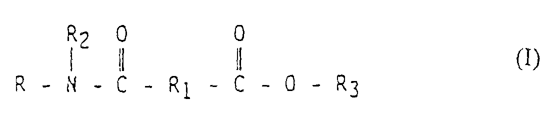 dans laquelle:
R est choisi parmi les alkyles en C₁ à C₂₀,
R₁ est choisi parmi les alkylènes en C₂ à C₁₄ et les alkénylènes en C₂ à C₁₄,
R₂ est choisi parmi un atome d'hydrogène et les groupes alkyle, aryle et aralkyle comportant 1 à 10 atomes de carbone, et
R₃ est choisi parmi les radicaux alkyles en C₁ à C₈;
avec au moins un équivalent d'un sel de métal alcalin par mole d'ester amide, à 20°C à 160°C, pour obtenir un sel d'acide amide; et
(B) acidification du sel d'acide amide par au moins 1,0 équivalent d'acide fort par mole de sel d'acide amide, à 20 à 160°C, pour obtenir un acide amide.

2. Un procédé selon la revendication 1, dans lequel l'étape d'hydrolyse (A) est effectuée jusqu'à ce que l'on ait transformé plus de 99,5 % de l'ester amide en le sel d'acide amide correspondant.

3. Un procédé selon l'une quelconque des revendications 1 à 2, comprenant en outre les étapes de:
(C) précipitation de l'acide amide à partir du milieu réactionnel aqueux de l'étape (B),
(D) isolement du précipité solide d'acide amide,
(E) lavage du précipité solide d'acide amide par une quantité suffisante d'un milieu aqueux pour éliminer pratiquement la totalité de l'alcanol résiduel de ce précipité, et
(F) séchage du précipité solide d'acide amide lavé à une température de 20 à 95°C.

4. Un procédé selon l'une quelconque des revendications 1 à 2, dans lequel l'étape (B) est effectuée à une température supérieure au point de fusion de l'acide amide, et comprenant en outre les étapes de:
(C) séparation de la phase liquide contenant l'acide amide à une température supérieure au point de fusion de l'acide amide, et
(D) lavage de la phase liquide contenant l'acide amide par une quantité suffisante d'un milieu aqueux pour éliminer pratiquement la totalité de l'alcanol résiduel.

5. Un procédé selon la revendication 4, comprenant en outre les étapes de:
(E) séchage et mise sous forme de flocons de l'acide amide, caractérisé en ce que les opérations de séchage et de mise en flocons peuvent être effectuées dans un ordre quelconque et en ce que l'étape de séchage réduit la teneur en eau de l'acide amide en-dessous de 2,0 % en poids.

6. Un procédé selon la revendication 5, dans lequel l'étape de séchage est effectuée avant l'étape de mise sous forme de flocons et que l'étape de séchage comprend un séchage de l'acide amide dans un évaporateur flash à 110 à 180°C.

7. Un procédé selon la revendication 6, dans lequel l'étape de séchage est effectuée dans un évaporateur flash à deux étapes, le premier étape opérant à 130-140°C sous 1,0 à 2,0 bar et pendant une durée suffisante pour réduire la teneur en eau de l'acide amide à 1,5 à 3,0 % en poids, et le second étape fonctionnant à une température et une pression plus basses que celles qui sont utilisées dans le premier éjape, et pendant une durée suffisante pour réduire encore la teneur en eau de l'acide amide. en-dessous de 0,5 % en poids.
